(19) Europäisches Patentamt
European Patent Office
Office européen des brevets

(11) Veröffentlichungsnummer: **0 300 381 B1**

(12) **EUROPÄISCHE PATENTSCHRIFT**

(45) Veröffentlichungstag der Patentschrift: **30.11.94**

(51) Int. Cl.5: **G01N 33/53**, //A01N43/00

(21) Anmeldenummer: **88111408.6**

(22) Anmeldetag: **15.07.88**

# (54) Immunologisches Nachweisverfahren für Herbizide.

(30) Priorität: **17.07.87 DE 3723726**

(43) Veröffentlichungstag der Anmeldung:
**25.01.89 Patentblatt 89/04**

(45) Bekanntmachung des Hinweises auf die Patenterteilung:
**30.11.94 Patentblatt 94/48**

(84) Benannte Vertragsstaaten:
**AT BE CH DE ES FR GB GR IT LI LU NL SE**

(56) Entgegenhaltungen:
**US-A- 4 530 786**

**JOURNAL OF AGRICULTURAL FOOD CHEMISTRY USA, Band 33, 1985; W.H. NEWSOME, Seiten 528-530/**

**CHEMOSPHERE, Band 14, 1985, Pergamon Press Ltd. (GB); S.J. HUBER, Seiten 1795-1803/**

**GEWÄSSERSCHUTZ-WASSER-ABWASSER, Band 106, 1989; W. PFEIFFER et al.,Seiten 400-413/**

**JOURNAL OF PLANT DISEASES AND PROTECTION, Band 92, Nr. 2, 1985, Eugen Ulmer GmbH & Co., Stuttgart (DE); S.J. HUBER et al., Seiten 147-156/**

(73) Patentinhaber: **DR. PFEIFFER BIOANALYTIK KG**
**Walchstadterstrasse 4**
**D-82266 Inning (DE)**

(72) Erfinder: **Pfeiffer, Wolfgang, Dr.**
**Im Harl 3**
**D-8133 Feldafing (DE)**
Erfinder: **Dittel, Rudolf H., Dr.**
**Bahnhofstrasse 18**
**D-8919 Utting (DE)**
Erfinder: **Mies, Wolfgang, Dr.**
**Bergstrasse 2**
**D-8035 Gauting (DE)**

(74) Vertreter: **Hansen, Bernd, Dr.rer.nat. et al**
**Hoffmann, Eitle & Partner**
**Patentanwälte**
**Postfach 81 04 20**
**D-81904 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 300 381 B1

## Beschreibung

Die Erfindung betrifft ein immunologisches Verfahren zum Nachweis von umweltrelevanten Stoffen, insbesondere von Pflanzenschutzmitteln, im Grund- bzw. Trinkwasser.

In letzter Zeit wurden alarmierende Berichte bekannt, aus denen eine Verunreinigung des Grundwassers, aus dem häufig Trinkwasser ohne großen Reinigungsaufwand gewonnen wird, mit Nitraten aus Düngemitteln, mit chlorierten Lösungsmitteln aus chemischen Reinigungen, mit Herbiziden (Pestiziden), Insektiziden und deren Abbauprodukten hervorgeht. Dabei stellen das Pflanzenschutzmittel Atrazin und seine Metabolite den größten Problemstoff im Trinkwasser dar.

Atrazin ist ein geruchsloses, weißes Pulver mit dem chemischen Namen 2-Chlor-4-ethylamino-6-isopropylamino-1,3,5-triazin und wird seit rund dreißig Jahren weltweit als Herbizid eingesetzt. Es unterbindet die Photosynthese und läßt damit "Unkraut" schnell verdorren. Atrazin wird insbesondere zur Unkrautvernichtung auf Maisfeldern eingesetzt, wobei 90% sämtlicher Maisanbauflächen in der BRD mit Atrazin behandelt werden, so daß Atrazin mit rund 1000 Jahrestonnen die Verbrauchsliste anführt. Neben dem Atrazin werden weitere Triazin-Herbizide eingesetzt, deren Vorkommen in Grund- und Oberflächenwässern nachgewiesen wurde. Tabelle 1 gibt einen Überblick über die wichtigsten Triazin-Herbizide.

Tab. 1: Triazin-Herbizide

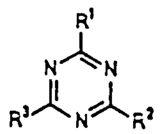

| | |
|---|---|
| Ametryn: | $R^1 = S-CH_3$, $R^2 = NH-CH(CH_3)_2$, $R^3 = NH-C_2H_5$ |
| Atraton: | $R^1 = O-CH_3$, $R^2 = NH-CH(CH_3)_2$, $R^3 = NH-C_2H_5$ |
| Atrazin: | $R^1 = Cl$, $R^2 = NH-C_2H_5$, $R^3 = NH-CH(CH_3)_2$ |
| Aziprotryn: | $R^1 = S-CH_3$, $R^2 = NH-CH(CH_3)_2$, $R^3 = N_3$ |
| Desmetryn: | $R^1 = S-CH_3$, $R^2 = NH-CH(CH_3)_2$, $R^3 = NH-CH_3$ |
| Dipropetryn: | $R^1 = S-C_2H_5$, $R^2 = R^3 = NH-CH(CH_3)_2$ |
| Methoprotryn: | $R^1 = S-CH_3$, $R^2 = NH-CH(CH_3)_2$, $R^3 = NH-C_3H_6-OCH_3$ |
| Prometryn: | $R^1 = S-CH_3$, $R^2 = R^3 = NH-C_3H_7$ |
| Propazin: | $R^1 = Cl$, $R^2 = R^3 = NH-C_3H_7$ |
| Simazin: | $R^1 = Cl$, $R^2 = R^3 = NH-C_2H_5$ |
| Terbumeton: | $R^1 = O-CH_3$, $R^2$, $R^3$ wie Terbutryn |
| Terbuthylazin: | $R^1 = Cl$, $R^2$, $R^3$ wie Terbutryn |
| Terbutryn: | $R^1 = S-CH_3$, $R^2 = NH-C(CH_3)_3$, $R^3 = NH-C_2H_5$ |

Lange Zeit ging man davon aus, daß diese Triazinverbindungen nach ihrer Anwendung nachhaltig zersetzt und an Bodenteilchen gebunden werden, so daß eine Gefährdung des Grundwassers ausgeschlossen ist. Es hat sich aber erwiesen, daß die Verbindungen recht stabil sind, un die Zeit, in der die Hälfte des Wirkstoffs im Boden abgebaut ist, zwischen zwei und fünf Monaten beträgt. In Sandböden sowie in lehm- und tonarmen Böden wird der Wirkstoff relativ leicht ins Grundwasser, in dem der Abbau noch länger dauert, ausgewaschen, so daß Triazinreste noch nach Jahren im Grundwasser auftauchen können.

Die Trinkwasserverordnung und die "EG-Rechtlinie über die Qualität von Wasser für den menschenlichen Gebrauch" legen Grenzwerte für noch tolerierbare Mengen dieser Stoffe im Trinkwasser fest. Für einzelne Substanzen beträgt dabei die Höchstgrenze 0,0001 mg/l (100 ng/l), und insgesamt darf die Summe dieser Stoffe die Konzentration von 0,0005 mg/l (500 ng/l) nicht überschreiten.

Für eine genaue Analytik sollte die Nachweisgrenze ein bis zwei Größenordnungen unter diesem Wert liegen. Die große Zahl der in Frage kommenden Stoffe und die niedrigen Grenzwerte stellen jedoch ein großes Problem für die chemische Analytik dar. Physikalisch-chemische Analysenmethoden (GC, GC-MS, HPLC) erfordern aufwendige Anreicherungsverfahren für eine Identifizierung und Quantifizierung von chemischen Stoffen und sind sehr kostspielig und zeitaufwendig. Zudem ermöglichen sie keine Aussage über die Toxizität der Verbindungen. Moderne biochemische Analyseverfahren werden durch immunologische Testverfahren, die sogenannten "Immunoassays", dargestellt, in denen Zellbestandteile als Testsubstanzen eingesetzt werden. Immunologische Analyseverfahren stellen hochempfindliche Schnelltests auf dem Sektor der Gewässeranalytik dar und gewährleisten häufig eine schnelles, kostengünstiges und effektives Umwelt-Monitoring.

Immunoassays sind hochempfindliche Testsysteme zur quantitativen Bestimmung von Substanzen auf der Grundlage der Antigen-Antikörper Reaktion. Dabei werden die Antikörper zunächst durch Immunisierung von Labortieren induziert und aus deren Seren oder aus Antikörper produzierenden Lymphozyten gewonnen, und über Affinitätssäulen gereinigt, die als Füllmaterial z.B. Agarose enthalten. Antikörper stellen eines der natürlichen Abwehrsysteme höherer, tierischer Lebewesen dar. Die Abwehrfunktion beruht darauf, daß spezifische Antikörper auf Grund einer natürlichen Infektion oder einer künstlichen Infektion beim Impfen induziert werden. Eine gewisse Molekülgröße ist hierbei zur Bildung von Antikörpern eine Voraussetzung. Soll gegen sehr kleine Moleküle, beispielsweise Pflanzenbehandlungsmittel, eine spezifische Antikörperbildung ausgelöst werden, so müssen diese (Haptene) vor der Immunisierung an ein hochmolekulares Trägermolekül, wie z.B. ein Protein (Hämocyanin, Rinderserumalbumin, Ovalbumin, Thyroglobulin, Polylysin und andere) gekoppelt werden.

Für den Nachweis eines chemischen Stoffes benutzt man nun die Antigen-Antikörper-Bindung, die bei Zugabe einer den chemischen Stoff als Antigen enthaltenden Probe zu dem Antikörper auftritt. Im klassischen Immunoassay konkurrieren die zu bestimmenden Antigene mit radioaktivmarkierten Antigenen gleicher Spezifität um die Bindungstellen am Antikörper. In neuerer Zeit hat der Enzym-Immunoassay (EIA) gegenüber dem Radioimmunoassay (RIA) an Bedeutung gewonnen. Bei diesem Verfahren wird das radioaktive Antigen durch ein Enzym-Antigen-Konjugat ersetzt, dass dann mit dem Probenantigen um die freien Bindungsplätze des Antikörpers konkurrieren kann. Bei diesem kompetitierenden EIA konkurriert eine vorgegebene Menge des enzym-markierten Antigens mit dem Probenantigen um die Bindungsstellen am Antikörper, die ihrerseits adsorptiv bzw. kovalent an eine Trägeroberfläche, z.B. Polystyroloberfläche, gebunden sind. Bei geringer Konzentration von Probenantigen wird viel Enzymtracer (hoher Substratumsatz), bei einer hohen Konzentration von Probeantigen wird dagegen nur wenig Enzymtracer (geringer Substratumsatz) gebunden. Die Inhibition der Enzymtracerbindung ist damit eine Funktion der Probenantigenkonzentration. Nach Abtrennung der ungebundenen Enzymtracerteile über einen Waschschritt läßt sich anhand der Substratumsetzung der Anteil des gebundenen, enzym-markierten Antigens und damit die Antigenkonzentration in der Probe ermitteln. Messgröße für die Bindung des Enzymtracers ist die Absorption der umgesetzten Substratmenge.

Mit der oben beschriebenen Technik werden spezifische Antikörper gegen verschiedene Pestizide induziert und in Immunoassays mit dem Ziel eines schnellen, hochspezifischen und damit kostengünstigen Nachweises dieses Problemstoffes entwickelt. Für das Atrazin wurde ein entsprechender Test entwickelt und patentiert (Dunbar et al. US-A-4 530 786). Dieser Test spricht auf den mit Chlor substituierten Wirkstoff aber nicht auf dessen Metabolite, die kein Chlor mehr enthalten, an. Der Nachweis dieser Metabolite wird aber durch die europäische Trinkwassergesetzgebung ebenfalls vorgeschrieben. Häufig liegt also der Fall vor, daß mit einem solchen Immunoassay eine eindeutige Identifizierung sowie Quantifizierung auf Grund von Kreuzreaktionen nicht möglich ist. Unter Kreuzreaktion versteht man das Phänomen, daß Antikörper gemeinsame Funktionalitäten in Molekülen unterschiedlicher Struktur erkennen und diese verschiedenen Moleküle einer Wirkungsklasse zuordnen, dabei aber nicht zwischen den einzelnen Molekülen unterscheiden können. Dieser Effekt tritt sehr deutlich bei kleinen Molekülen (Antigenen) auf, da diese an eine Trägermatrix (Protein) gebunden werden müssen, und damit nur die der Trägermatrix abgewandte Seite, und nicht das gesamte Molekül, als Erkennungsregion wirkt. Moleküle mit ähnlicher Struktur dieser abgewandten Seite lassen sich dann in Immunoassays häufig nicht unterscheiden. Dies wird im allgemeinen als Nachteil angesehen. Im vorliegenden Fall ist hingegen dieser Effekt der Kreuzreaktion gerade erwünscht, da nicht nur das speziell für die Herstellung eines Antikörpers verwendete Antigen, sondern auch Verbindungen mit ähnlicher chemischer Struktur bzw. mit ähnlicher biologischer Wirksamkeit wie das verwendete Antigen erfaßt werden können (vgl. Tabelle 2). Mit einem Test lassen sich unter bewußter Ausnutzung der Kreuzreaktionen unterschiedliche, jeweils zu einer Wirkklasse gehörenden Stoffe erfassen (vgl. Tabelle 3). Dies ist sehr wertvoll bei der toxischen Beurteilung bislang unerforschter Verbindungen. Tierversuche und Bioassays lassen sich damit auf das Notwendigste reduzieren, da das Gefahrenpotential ökotoxikologisch bisher unerforschter Verbindungen nun schon im Vorfeld durch die oben beschriebene Wirkklassenzuordnung erkennbar wird.

Der Erfindung liegt die Aufgabe zu Grunde, ein immunologisches Nachweisverfahren bereitzustellen, mit dem Triazin-Herbizide und Verbindungen mit chemisch ähnlicher Struktur bzw. mit biologisch ähnlicher Wirkungsweise als zu einer Wirkklasse gehörig erfaßt werden, mit dem es andererseits aber auch möglich ist, die einzelnen chemischen Stoffe auch in komplizierten Gemischen eindeutig zu identifizieren.

Die vorstehende Aufgabe wird gelöst durch ein immunologisches Verfahren zur Bestimmung von Verbindungen der folgenden Formel:

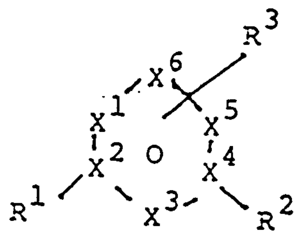

(I)

worin bedeuten:

$X^1$, $X^3$, $X^5$ Kohlenstoff oder Stickstoff;

$X^2$, $X^4$, $X^5$ Kohlenstoff;

$R^1$ die Gruppe $HN(C_nH_{2n+1})$, wobei n die Zahl von 0 bis 8 darstellt;

$N(C_mH_{2m+1})_2$, wobei m die Zahl 1 bis 8 bedeutet;

$HN\text{-}C_nH_{2n}Y$, wobei Y eine Cyano-, Amino- oder COOH-Gruppe bedeutet, und n die Zahl 1 bis 8 darstellt;

$C_xH_{2x}Z$, wobei Z eine Cyano-, Amino- oder COOH-Gruppe bedeutet, und x die Zahl 1 bis 8 darstellt;

Azid, Halogen, eine SH-Gruppe, eine OH-Gruppe oder eine $HN\text{-}C_6H_4Cl$-Gruppe;

$R^2$ eine Gruppe $HNC_qH_{2q+1}$, wobei q eine Zahl von 0 bis 8 darstellt;

$N(C_rH_{2r+1})_2$, wobei r die Zahl 1 bis 8 bedeutet;

$HN\text{-}C_qH_{2q}Y$, wobei Y eine Cyano-, Amino-, Azido- oder COOH-Gruppe bedeutet und q die Zahl 1 bis 8 darstellt;

$N(C_rH_{2r+1})Z$, wobei Z eine Cyano-, Amino-, Azido- oder COOH-Gruppe oder eine $C_qH_{2q}$-Gruppe bedeutet, und r eine Zahl von 1 bis 8 und q eine Zahl von 1 bis 8 darstellt;

Azid, Halogen, eine SH-Gruppe oder eine OH-Gruppe;

$R^3$ Halogen; $SC_nH_{2n+1}$, wobei n eine Zahl von 0 bis 8 darstellt; $OC_nH_{2n+1}$, wobei n eine Zahl von 0 bis 8 darstellt; eine Cyano-, Carboxyl-, Amino- oder CHO-Gruppe, wobei sich der Substituent $R^3$ entweder in meta-Stellung zu $R^1$ und $R^2$ oder in ortho- bzw. para-Stellung zu $R^1$ und $R^2$ befinden kann, das dadurch gekennzeichnet ist, daß durch Koppeln einer vorstehend genannten Verbindung (I) (Antigen) über den Substituenten $R^1$ oder $R^2$ oder $R^3$ an eine Matrix jeweils nur ein Teil des Moleküls der Verbindung (I) exponiert ist, und im folgenden durch Immunisierung von Labortieren ein bevorzugt auf den jeweils unterschiedlichen exponierten Teil des Moleküls der Verbindung (I) ansprechender Antikörper A, B oder C gewonnen wird, und zum immunologischen Nachweis der Verbindung (I) eine Probe, die diese angeblich enthält, mindestens mit zwei der vorstehend genannten Antikörper A, B oder C in Versuchsserien jeweils separat umgesetzt wird, und aufgrund der vorliegenden Antigen-Antikörper-Bindung der jeweiligen Versuchsserien eine qualitative und quantitative Bestimmung der Verbindung (I) erfolgt.

Die Probe mit der zu bestimmenden Substanz wird dabei jeweils separat mit dem Antikörper umgesetzt. Man kann hierbei so verfahren, daß die zu untersuchenden Lösungen in die mit den entsprechenden Antikörpern beschichteten Reaktionsgefässe bzw. Kavitäten einer Mikrotiterplatte pipettiert werden.

Eine zweite Ausführungsform des erfindungsgemäßen Verfahrens zur immunologischen Bestimmung von Verbindungen der Formel (I) ist dadurch gekennzeichnet, daß durch Koppeln einer vorstehend genannten Verbindung (I) (Antigen) über den Substituenten $R^1$ oder $R^2$ oder $R^3$ an eine Matrix jeweils nur ein Teil des Moleküls der Verbindung (I) exponiert ist, und im folgenden durch Immunisierung von Labortieren ein bevorzugt auf diesen exponierten Teil des Moleküls der Verbindung ansprechender Antikörper A, B oder C gewonnen wird, und zum immunologischen Nachweis der Verbindung (I) eine Probe, die diese angeblich enthält, mit mindestens zwei der vorstehend genannten Antikörper A, B oder C in Versuchsserien mit unterschiedlicher Reihenfolge der genannten Antikörper umgesetzt wird, wobei die Probe zunächst mit dem ersten Antikörper der jeweiligen Versuchsserie umgesetzt wird, dann der nicht reagierte Überstand abgetrennt und dieser jeweils mit dem nächsten Antikörper der Versuchsserie umgesetzt wird, und aufgrund der vorliegenden Antigen-Antikörper-Bindung der jeweiligen Versuchsserien eine qualitative und quantitative Bestimmung der Verbindung (I) erfolgt.

Eine bevorzugte Anwendung des erfindungsgemässen Verfahrens gemäß Anspruch 2 besteht darin, daß für die immunologische Bestimmung drei Antikörper A, B und C in Versuchsserien mit folgender Reihenfolge eingesetzt werden:

1. Versuchsserie: A B C
2. Versuchsserie: B C A
3. Versuchsserie: C A B.

In einer bevorzugten Ausführungsform des erfindungsgemässen Verfahrens stellt die Verbindung der Formel (I) einen meta-substituierten, bis zu drei Stickstoffatome enthaltenden aromatischen Sechsring dar. Besonders vorteilhaft sind hierbei Pyridine, Pyrimidine oder Triazine.

In speziellen Fällen kann es sich bei der Anwendung des erfindungsgemässen Verfahrens als günstig erweisen, monoklonale Antikörper bei der Aufbereitung der aus Labortieren erhaltenen Seren bzw. Antikörper produzierenden Lymphozyten mit Hilfe der Hybridomtechnik zu gewinnen, und diese als Antikörper in dem Nachweisverfahren einzusetzen.

Das immunologische Testverfahren der vorliegenden Erfindung ermöglicht es, neben der Verbindung (I) durch Kreuzreaktivität auch strukturell ähnliche Verbindungen, die dieser Verbindungsklasse angehören, und auch davon strukturell verschiedene Verbindungen, die aber die gleiche biologische Wirksamkeit und ähnliches Bindeverhalten zeigen, zu erfassen.

Die immunologische Nachweisbestimmung der Antigen-Antikörper Reaktion erfolgt bei dem erfindungsgemässen Verfahren vorteilhafterweise mit einem entsprechenden Antigen, das radioaktiv markiert oder an ein Enzym, an eine fluoreszierende Substanz oder an eine elektrische Signale abgebende Substanz (Biosensor) gebunden ist. Probenantigen und markiertes bzw. gebundenes Antigen (Nachweismittel) läßt man um die Bindungsstellen des Antikörpers konkurrieren, wäscht dann das nicht an den Antikörper gebundene, freie Nachweismittel aus und kann anschließend durch Messung der radioaktiven Strahlung, der Absorption, der Fluoreszensstrahlung oder des elektrischen Signals die Menge an gebundenem Nachweismittel und damit die Konzentration des Antigens in der Lösung bestimmen.

Das vorliegende, erfindungsgemäße immunologische Nachweisverfahren ermöglicht es, Stoffe einer bestimmten Wirkklasse zu erfassen, und gleichzeitig die verschiedenen Stoffe dieser Wirkklasse einzeln zu identifizieren. Es wird ein Testverfahren bereitgestellt, das schnell, hochspezifisch und kostengünstig arbeitet und das hohe Probeaufkommen in der Umweltanalytik bewältigen kann, was bei herkömmlicher Analytik trotz Automation nicht möglich ist. Das Verfahren ist so konzipiert, daß bislang in ihrer Toxizität noch nicht erforschte Verbindungen durch die Zuordnung zu Wirkklassen ökologisch beurteilt werden können.

Die vorliegende Erfindung wird anhand der folgenden Beispiele näher erläutert.

Beispiel 1

Im folgenden wird die Herstellung drei verschiedener Antikörper A, B und C, die jeweils bevorzugt gegen eine Seite des Herbizids Atrazin, eine bevorzugte Verbindung aus der Reihe der Verbindungen mit der Formel (I), gerichtet sind, und eine Durchführungsmethode des erfindungsgemäßen, immunologischen Testverfahrens beschrieben.

Für die Herstellung eines Antikörpers A, der bevorzugt gegen die Seite

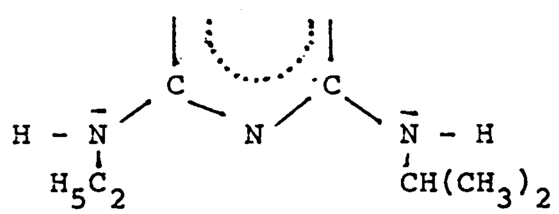

(II)

des Atrazinmoleküls gerichtet ist, wird zunächst die Methylthioether-Gruppe des Triazins Ametryn mit Persäure sulfoxidiert und anschließend das modifizierte Ametryn an Rinderserumalbumin direkt gekoppelt. Analog dazu bindet man für die Entwicklung eines Antikörpers B, der preferentiel gegen die Seite

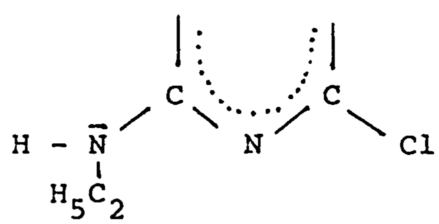

(III)

des Atrazinmoleküls gerichtet ist, eine der Ethylamino-Gruppen des Triazins simazin über das Carbodiimid-Verfahren an das Protein. Schließlich kann ein Antikörper C, der bevorzugt auf die Seite

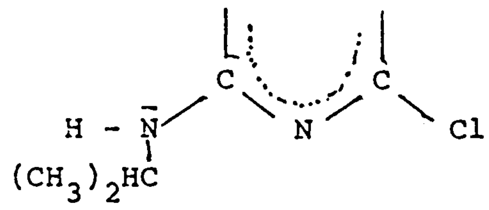

(IV)

des Atrazins anspricht, hergestellt werden, indem man das Herbizid Propazin über eine der Isopropylamino-Gruppen an die Trägermatrix koppelt.

Die oben hergestellten Konjugate werden zur Erzeugung der Antikörper A, B und C Labortieren (Kanninchen) geimpft, und diese dann aus den Seren der immunisierten Tieren nach bekannten Verfahren bzw. durch Affinitätschromatographie mittels Substituenten nach Formel (I) gewonnen.

Die erhaltenen Antikörper werden an der Polystyroloberfläche von Mikrotiterkavitäten adsorbiert. Dazu löst man die Antikörper in Carbonatpuffer ($Na_2CO_3$/$NaHCO_3$;50 mmol/liter; pH 9,6) und pipettiert jeweils 0,25 ml der erhaltenen Lösung in einzelne Kavitäten der Mikrotiterplatte, inkubiert über Nacht bei Raumtemperatur und wäscht am nächsten Tag mit TBS-Puffer. Die gut ausgeschlagenen Platten werden dann trocken bei -20 °C gelagert. Zur Analyse füllt man 180 ul der zu untersuchenden, wässrigen Probe (Konzentration des zu untersuchenden Stoffes zwischen 1 µg/l und 200 mg/l) in die Kavitäten ein und gibt jeweils 50 µl Enzymtracer, ein Konjugat aus alkalischer Phosphatase und einem Triazin, das je nach Art des Antikörpers ausgewählt ist, hinzu.

Wird der Nachweis mit dem Antikörper A durchgeführt, so wird Ametryn an das Enzym gekoppelt, liegt hingegen der Antikörper B bzw. der Antikörper C vor, so verwendet man Simazin bzw. Propazin.

Durch kurzes, horizontales Schütteln der Mikrotiterplatte werden die Lösungen durchmischt. Nach einer weiteren Inkubationszeit von 1 Stunde bei 20 °C entfernt man durch Waschen mit TBS-Puffer (50 mmol/l Tris-(Hydroxymethyl)-Aminomethan, 1 mmol/l $MgCl_2$, 50 mmol/l NaCl, pH 7,8 mit HCl, 0,05 % Polyoxyethylensorbitanmonooleat) alle nicht an Antikörperstellen gebundenen Moleküle. Zur quantitativen Bestimmung des gebunden Enzymtracers kommen jeweils 125 µl Phosphatase-Substratlösung (1 mg p-Nitrophenylphosphat pro ml Carbonat-Puffer) in die einzelnen Kavitäten. Die Enzymreaktion erfolgt bei 20 °C, kann aber durch Inkubation bei höherer Temperatur beschleunigt werden. Die Absorption der Lösung in den einzelnen Kavitäten wird mit einem Senkrechtstrahlphotometer bei der Wellenlänge 405 nm gemessen.

Will man in den Bereich höherer Empfindlichkeiten vordringen, so empfiehlt sich eine Übernachtinkubation bei 4 °C. Hierbei ist dann allerdings zu berücksichtigen, daß ohne besondere Anforderungen an die Reagenz-, Geräte- und Raumreinheit reproduzierbare Ergebnisse im ppt-Bereich nicht zu erzielen sind.

Die Berechnung der Probenantigenkonzentration basiert auf dem folgenden Hintergrund. Der Enzymtracer bindet zunächst an die freien Antikörperbindestellen, die nach Inkubation mit dem Probenantigen übrig bleiben. So wird in Gegenwart hoher Konzentrationen von Probenantigen nur wenig Enzymtracer gebunden, bei geringen Konzentrationen von Probenantigen dagegen viel Enzymtracer. Die Menge des gebundenen Enzymtracers stellt damit eine Funktion der Probenantigenkonzentration dar. Als Messgröße für die Bindung des Enzymtracers dient die Absorption der umgesetzten Substratmenge (Farbreaktion von farblos zu gelb).

Berechnung:

Absorptionsverhältnis von unbekannter Probe ($B = A_B - A_{uB}$) und Nullprobe ($BO = A_{BO} - A_{uB}$):

$$B / BO = (A_B - A_{uB}) / (A_{BO} - A_{uB})$$

(B/BO): Verhältnis der Enzymtracerbindung in Gegenwart eines Antigenes (B) im Verhältnis zur Enzymtracerbindung in Abwesenheit eines Antigenes (BO).

$A_{BO}$: Absorption (405 nm) als Maß für die Enzymtracerbindung in Abwesenheit von Antigen (Herbizid).

$A_B$: Absorption (405 nm) als Maß für die Enzymtracerbindung in Gegenwart einer unbekannten Konzentration von Antigen (Herbizid) in der Probe.

$A_{UB}$: Absorption (405 nm) als Maß für die Enzymtracerbindung in Abwesenheit von wandständigen Antikörpern.

Trägt man B/BO auf der y-Achse gegen den Logarithmus der Herbizidkonzentration auf, so ergibt sich ein sigmoider Kurvenverlauf. Zur Linearisierung zwecks besserer Handhabung wurde vorgeschlagen, die y-Achse ebenfalls zu logarithmieren (logit/log-Transformation). Für die Auswertung erwies es sich jedoch problemnäher, den Grad der Inhibition (1 - B/BO) über das Integral der Normalverteilung zu transformieren. In der Praxis trägt man hierzu die prozentuale Inhibition (100* (1-B/BO)) an Stelle der Häufigkeit in das Wahrscheinlichkeitspapier ein. Hierdurch wird die sigmoide Kurve linearisiert.

Das Nachweisverfahren für das Probenantigen kann so geführt werden, daß man in den einzelnen Versuchsserien das Probenantigen mit jedem der eingesetzten Antikörper separat oder hintereinander umsetzt. Bei der letzteren Methode ergibt sich, daß man bei jeder Versuchsserie gleiche Inhibitionen bei einer Nachweisbestimmung beobachtet, wenn die exponierten Teile, auf die die verschiedenen Antikörper bevorzugt gerichtet sind, in verschiedenen Molekülen vorliegen. Sind jedoch mindestens zwei exponierte Seiten in einem Molekül enthalten, kommt es zu Abweichungen. Dies ist dadurch zu erklären, daß ein Molekül, das zwei der eingangs genannten exponierten Seiten besitzt, durch die Antikörperbindung an der einen wie auch an der anderen Seite aus der Lösung entfernt wird und nach Transfer der Lösung in die nächste Kavität nicht mehr zur Bindung zur Verfügung steht. Führt man dies weiter für alle Bindemöglichkeiten aus, so ergibt sich folgende Inhibitionsmatrix (Tabelle 2), aus der sich die Grundstrukturen in einem Multiverfahren ableiten lassen.

Tab. 2

| Inhibitionsmatrix | | | | | | | | | |
|---|---|---|---|---|---|---|---|---|---|
| 1. Versuchsserie: | | | 2. Versuchsserie: | | | 3. Versuchsserie: | | | |
| A | B | C | B | C | A | C | A | B | |
| + | + | + | + | + | + | + | + | + | alle Seiten (II), (III), (IV) liegen unabhängig vor (3 Komponenten) |
| + | - | - | + | - | - | + | - | - | alle Seiten (II), (III), (IV) sind in einem Molekül enthalten (1 Komponente) |
| + | - | + | + | + | - | + | + | - | die Seiten (II) und (III) liegen in einem Molekül vor (2 Komponenten) |
| + | + | - | + | + | - | + | - | + | die Seiten (II) und (IV) liegen in einem Molekül vor (2 Komponenten) |
| + | + | - | + | - | + | + | + | - | die Seiten (III) und (IV) liegen in einem Molekül vor (2 Komponenten) |
| + = Inhibition der Enzymtracerbindung<br>- = keine Inhibition der Enzymtracerbindung | | | | | | | | | |

Die hier aufgeführte Inhibitionsmatrix läßt sich durch Verwendung weiterer Antikörper leicht über C hinaus erweitern, um auch noch kompliziertere Moleküle bzw. Molekülgemische zu analysieren.

Beispiel 2

Es wurde die Kreuzreaktivität von Triazinen mit Vertretern der in Tabelle 3 aufgeführten Substanzklassen untersucht, um diese Verbindungen hinsichtlich ihrer Wirkklassenzugehörigkeit einordnen zu können. Die Immunisierung erfolgte in der Weise, daß spezifisch die Alkyl-, Isopropyl- und Ethylreste exponiert wurden, um eine hohe Spezifität für Atrazin zu erlangen. In der folgenden Tabelle sind die Inhibitionswerte (%) der wichtigen Vertreter einzelner Substanzklassen aufgeführt. Eine starke Bindung eines Antigens an ein Antikörper bewirkt eine ebenso starke Inhibition des enzymatischen Nachweissystems. Aus der Stärke der Inhibition läßt sich der Grad der Wechselwirkung zwischen Antikörper und Antigen erkennen.

Tab. 3

| Kreuzaktivität verschiedner Herbizide bei einer Konzentration von 2 ppb | | |
|---|---|---|
| Substanzklasse: | Verbindung: | Inhibition (%): |
| Triazine | Atrazin | 6,0 |
| | Hydroxy-3 isopropyl-5-ethyltriazin | 5,3 |
| | Ametryn | 21,4 |
| | Desethylametryn | 16,8 |
| | Desisopropylametryn | 16,5 |
| Anilide | Metazachlor | 15,2 |
| Harnstoffe | Metabenzthiazuron | 8,3 |
| Trifluorbenzimidazole | Fenazaflor | 4,4 |
| Aminotriazine | Metribuzin | 6,7 |
| Uracile | Bromazil | 11,1 |

Die in der Tabelle 3 angegebenen Inhibitionswerte zeigen, daß in dem angegebenen Konzentrationsbereich (2 ppb) die Selektivität der Antikörper nicht so stark ausgeprägt ist, daß aber die aufgeführten Verbindungen, die ebenfalls auf das Photosystem II wirken, in einem immunologischen Nachweisverfahren gleichsam erfaßt werden können. Bei der erfindungsgemässen Konzeption des Immunoassays ist es möglich, durch bewußte Ausnutzung der Kreuzreaktivitäten Rückschlüsse auf die Struktur der einzelnen in einem Gemisch vorliegenden Stoffe zu schließen (vergleiche Beispiel 1).

**Patentansprüche**

**1.** Immunologisches Verfahren zur Bestimmung von Verbindungen der folgenden Formel:

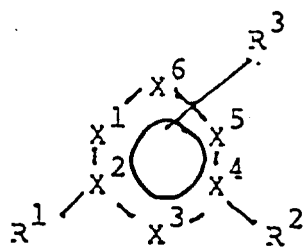

(I)

worin bedeuten:
$X^1$, $X^3$, $X^5$ Kohlenstoff oder Stickstoff;
$X^2$, $X^4$, $X^5$ Kohlenstoff;
$R^1$ die Gruppe $HN(C_nH_{2n+1})$, wobei n die Zahl von 0 bis 8 darstellt;
$N(C_mH_{2m+1})_2$, wobei m die Zahl 1 bis 8 bedeutet;
$HN\text{-}C_nH_{2n}Y$, wobei Y eine Cyano-, Amino- oder COOH-Gruppe bedeutet, und n die Zahl 1 bis 8 darstellt;
$C_xH_{2x}Z$, wobei Z eine Cyano-, Amino- oder COOH-Gruppe bedeutet, und x die Zahl 1 bis 8 darstellt;
Azid, Halogen, eine SH-Gruppe, eine OH-Gruppe oder eine $HN\text{-}C_6H_4Cl$-Gruppe;
$R^2$ eine Gruppe $HNC_qH_{2q+1}$, wobei q eine Zahl von 0 bis 8 darstellt;
$N(C_rH_{2r+1})_2$, wobei r die Zahl 1 bis 8 bedeutet;
$HN\text{-}C_qH_{2q}Y$, wobei Y eine Cyano-, Amino-, Azido- oder COOH-Gruppe bedeutet und q die Zahl 1 bis 8 darstellt;
$N(C_rH_{2r+1})Z$, wobei Z eine Cyano-, Amino-, Azido- oder COOH-Gruppe oder eine $C_qH_{2q}$-Gruppe bedeutet,
und r eine Zahl von 1 bis 8 und q eine Zahl von 1 bis 8 darstellt;
Azid, Halogen, eine SH-Gruppe oder eine OH-Gruppe;
$R^3$ Halogen; $SC_nH_{2n+1}$, wobei n eine Zahl von 0 bis 8 darstellt; $OC_nH_{2n+1}$, wobei n eine Zahl von 0 bis 8 darstellt; eine Cyano-, Carboxyl-, Amino- oder CHO-Gruppe, wobei sich der Substituent $R^3$ entweder in meta-Stellung zu $R^1$ und $R^2$ oder in ortho- bzw. para-Stellung zu $R^1$ und $R^2$ befinden kann,

dadurch **gekennzeichnet,** dass durch Koppeln einer vorstehend genannten Verbindung (I) (Antigen) über den Substituenten $R^1$ oder $R^2$ oder $R^3$ an eine Matrix jeweils nur ein Teil des Moleküls der Verbindung (I) exponiert ist, und im folgenden durch Immunisierung von Labortieren ein bevorzugt auf den jeweils unterschiedlichen exponierten Teil des Moleküls der Verbindung ansprechender Antikörper A, B oder C gewonnen wird, und zum immunologischen Nachweis der Verbindung (I) eine Probe, die diese angeblich enthält, mindestens mit zwei der vorstehend genannten Antikörper A, B oder C in Versuchsserien jeweils separat umgesetzt wird, und aufgrund der vorliegenden Antigen-Antikörper-Bindung der jeweiligen Versuchsserien eine qualitative und quantitative Bestimmung der Verbindung (I) durchgeführt wird.

**2.** Immunologisches Verfahren zur Bestimmung von Verbindungen der folgenden Formel:

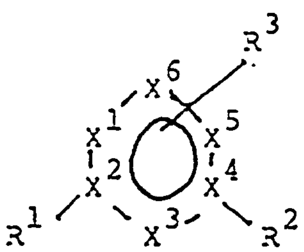

(I)

worin $X^1$, $X^2$, $X^3$, $X^4$, $X^5$, $X^5$, $R^1$, $R^2$ und $R^3$ dieselben Bedeutungen wie in Anspruch 1 haben,
dadurch **gekennzeichnet,** dass durch Koppeln einer vorstehend genannten Verbindung (I) (Antigen) über den Substituenten $R^1$ oder $R^2$ oder $R^3$ an eine Matrix jeweils nur ein Teil des Moleküls der Verbindung (I) exponiert ist, und im folgenden durch Immunisierung von Labortieren ein bevorzugt auf diesen exponierten Teil des Moleküls der Verbindung ansprechender Antikörper A, B oder C gewonnen wird, und zum immunologischen Nachweis der Verbindung (I) eine Probe, die diese angeblich enthält, mindestens mit zwei der vorstehend genannten Antikörper A, B oder C in Versuchsserien mit unterschiedlicher Reihenfolge der genannten Antikörper umgesetzt wird, wobei die Probe zunächst mit dem ersten Antikörper der jeweiligen Versuchsserie umgesetzt wird, dann der nicht reagierte Überstand abgetrennt und dieser jeweils mit dem nächsten Antikörper der Versuchsserie umgesetzt wird, und aufgrund der vorliegenden Antigen-Antikörper-Bindung der jeweiligen Versuchsserien eine qualitative und quantitative Bestimmung der Verbindung (I) durchgeführt wird.

**3.** Verfahren nach Anspruch 1 oder 2, dadurch **gekennzeichnet,** dass die Verbindung der Formel (I) einen meta-substituierten, bis zu drei Stickstoffatome enthaltenden aromatischen Sechsring darstellt.

**4.** Verfahren nach Anspruch 3, dadurch **gekennzeichnet,** dass die Verbindung (I) ein Pyridin, Pyrimidin oder Triazin darstellt.

**5.** Verfahren nach Anspruch 2 , dadurch **gekennzeichnet**, dass für die immunologische Bestimmung drei Antikörper A, B und C in Versuchsserien mit folgender Reihenfolge eingesetzt werden:
1. Versuchsserie: A B C
2. Versuchsserie: B C A
3. Versuchsserie: C A B.

**6.** Verfahren nach einem oder mehreren der vorangehenden Ansprüche, dadurch **gekennzeichnet,** dass die Antikörper monoklonale Antikörper darstellen.

**7.** Verfahren nach einem oder mehreren der vorangehenden Ansprüche, dadurch **gekennzeichnet,** dass neben der Verbindung (I) durch Kreuzreaktivität auch strukturell ähnliche Verbindungen, die dieser Verbindungsklasse angehören, und auch davon strukturell verschiedene Verbindungen, die aber die gleiche biologische Wirksamkeit und ähnliches Bindeverhalten zeigen umfasst werden.

**8.** Verfahren nach einem oder mehreren der vorangehenden Ansprüche, dadurch **gekennzeichnet,** dass die immunologische Nachweisbestimmung mit Hilfe eines radioaktiven Isotops, eines Enzyms, einer fluoreszierenden Substanz oder einer, ein elektrisches Signal abgebenden Substanz erfolgt.

## Claims

1. Immunological process for assaying compounds of the following formula:

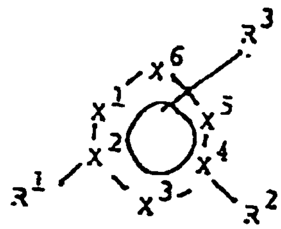

(I)

wherein:
$X^1$, $X^3$, $X^5$ denote carbon or nitrogen;
$X^2$, $X^4$, $X^5$ denote carbon;
$R^1$ denotes the group $HN(C_nH_{2n+1})$, wherein n is the number from 0 to 8;
$N(C_mH_{2m+1})_2$, wherein m denotes the number 1 to 8;
$HN-C_nH_{2n}Y$, wherein Y denotes a cyano, amino or COOH group, and n is the number 1 to 8;
$C_xH_{2x}Z$, wherein Z denotes a cyano, amino or COOH group, and x is the number 1 to 8;
azide, halogen, an SH group, an OH group or an $HN-C_6H_4Cl$ group;
$R^2$ denotes a group $HNC_qH_{2q+1}$, wherein q is a number from 0 to 8;
$N(C_rH_{2r+1})_2$, wherein r denotes the number 1 to 8;
$HN-C_qH_{2q}Y$, wherein Y denotes a cyano, amino, azido or COOH group and q is the number 1 to 8;
$N(C_rH_{2r+1})Z$, wherein Z denotes a cyano, amino, azido or COOH group or a $C_qH_{2q}$ group, and r is a number from 1 to 8 and q is a number from 1 to 8;
azide, halogen, an SH group or an OH group;
$R^3$ denotes halogen; $SC_nH_{2n+1}$, wherein n is a number from 0 to 8; $OC_nH_{2n+1}$, wherein n is a number from 0 to 8; a cyano, carboxyl, amino or CHO group, it being possible for substituent $R^3$ to be either in the meta position with respect to $R^1$ and $R^2$ or in the ortho position or para position with respect to $R^1$ and $R^2$, characterised in that by coupling a compound (I) mentioned above (antigen) to a matrix via substituents $R^1$ or $R^2$ or $R^3$, in each case only a part of the molecule of compound (I) is exposed, and an antibody A, B or C preferably responding to the exposed part of the molecule of the compound, which is different in each case, is obtained subsequently by immunising laboratory animals, and for immunological identification of compound (I), a sample, supposedly containing the latter, is reacted separately in each case with at least two of the antibodies A, B or C mentioned above in test series, and a qualitative and quantitative assay of compound (I) is carried out on the basis of the existing antigen-antibody bond of the particular test series.

2. Immunological process for assaying compounds of the following formula:

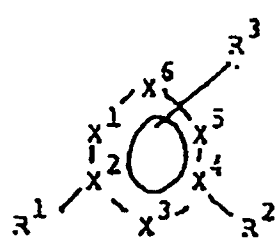

(I)

wherein $X^1$, $X^2$, $X^3$, $X^4$, $X^5$, $X^5$, $R^1$, $R^2$ and $R^3$ have the same meanings as in claim 1, characterised in that by coupling a compound (I) mentioned above (antigen) to a matrix via substituents $R^1$ or $R^2$ or $R^3$, in each case only a part of the molecule of compound (I) is exposed, and an antibody A, B or C preferably responding to this exposed part of the molecule of the compound is obtained subsequently by immunising laboratory animals, and for immunological identification of compound (I), a sample, supposedly containing the latter, is reacted with at least two of the antibodies A, B or C mentioned above in test series in a different sequence for the antibodies mentioned, the sample being initially

reacted with the first antibody of the particular test series, then the unreacted supernatant is separated off and the latter is reacted in each case with the next antibody of the test series, and a qualitative and quantitative assay of compound (I) is carried out on the basis of the existing antigen-antibody bond of the particular test series.

**3.** Process according to claim 1 or 2, characterised in that the compound of the formula (I) is a meta-substituted aromatic six-membered ring containing up to three nitrogen atoms.

**4.** Process according to claim 3, characterised in that the compound (I) is a pyridine, pyrimidine or triazine.

**5.** Process according to claim 2, characterised in that for immunological assay three antibodies A, B and C are used in test series in the following sequence:
1. test series: A B C
2. test series: B C A
3. test series: C A B.

**6.** Process according to one or more of the preceding claims, characterised in that the antibodies are monoclonal antibodies.

**7.** Process according to one or more of the preceding claims, characterised in that in addition to compound (I), due to cross-reactivity, structurally similar compounds which belong to this class of compounds, and also compounds which are structurally different from them but which show the same biological effectiveness and similar bonding behaviour, are also included.

**8.** Process according to one or more of the preceding claims, characterised in that the immunological identification assay is carried out with the aid of a radioactive isotope, an enzyme, a fluorescent substance or a substance emitting an electrical signal.

## Revendications

**1.** Procédé immunologique pour le dosage de composés répondant à la formule suivante :

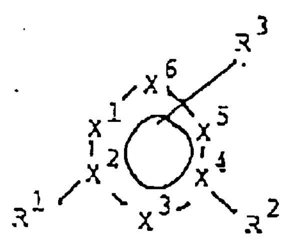

(I)

dans laquelle
$X^1$, $X^3$, $X^5$ désignent des atomes de carbone ou d'azote;
$X^2$, $X^4$, $X^5$ des atomes de carbone;
$R^1$ le groupe $HN(C_nH_{2n+1})$ n désignant un nombre de 0 à 8;
$N(C_mH_{2m+1})_2$, m désignant un nombre de 1 à 8;
$HN-C_nH_{2n}Y$, Y désignant un groupe cyano, amino ou COOH, et n un nombre de 1 à 8;
$C_xH_{2x}Z$, Z désignant un groupe cyano, amino ou COOH et x un nombre de 1 à 8;
un groupe azide, un atome d'halogène, un groupe SH, un groupe OH ou un groupe $HN-C_6H_4Cl$;
$R^2$ désigne un groupe $HNC_qH_{2q+1}$, q étant un nombre de 0 à 8;
$N(C_rH_{2r+1})_2$, r désignant un nombre de 1 à 8;
$HN-C_qH_{2q}Y$, Y désignant un groupe cyano, amino, azido ou COOH, et q un nombre de 1 à 8;
$N(C_rH_{2r+1})Z$, Z désignant un groupe cyano, amino, azido ou COOH, ou un groupe $C_qH_{2q}$, et r un nombre de 1 à 8, et q un nombre de 1 à 8;
un groupe azide, un atome d'halogène, un groupe SH ou un groupe OH;
$R^3$ désigne un atome d'halogène; $SC_nH_{2n+1}$, n désignant un nombre de 0 à 8;

$OC_nH_{2n+1}$, n désignant un nombre de 0 à 8; un groupe cyano, carboxyle, amino ou CHO, le substituant $R^3$ pouvant se trouver soit en position méta par rapport à $R^1$ et $R^2$, soit en position ortho ou para par rapport à $R^1$ ou $R^2$,
caractérisé en ce que par couplage d'un composé (I) précité (antigène) sur les substituants $R^1$ ou $R^2$ ou $R^3$ à une matrice, seule une partie de la molécule du composé (I) est exposée à chaque fois, et l'on obtient dans ce qui suit par immunisation d'animaux de laboratoire un anticorps A, B ou C correspondant de préférence à la partie différente à chaque fois de la molécule du composé, et on fait réagir à chaque fois séparément, pour la détection immunologique du composé (I), un échantillon qui est supposé les contenir, au moins avec deux des anticorps A, B ou C précités dans les séries d'essais, et on effectue, sur la base de la liaison antigène-anticorps existante de chaque série d'essais une détermination qualitative et quantitative du composé (I).

2. Procédé immunologique pour la détermination de composés répondant à la formule :

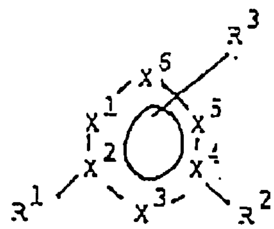

(I)

dans laquelle $X^1$, $X^2$, $X^3$, $X^4$, $X^5$, $X^5$, $R^1$, $R^2$ et $R^3$ possèdent les mêmes significations que dans la revendication 1,
caractérisé en ce que par couplage d'un composé (I) précité (antigène) sur les substituants $R^1$ ou $R^2$ ou $R^3$, à une matrice, à chaque fois une partie seulement de la molécule du composé (I) est exposée, et on obtient dans ce qui suit par immunisation d'animaux de laboratoire un anticorps A, B ou C correspondant à cette partie exposée de la molécule du composé, et on fait réagir un échantillon qui est supposé les contenir, ou au moins avec deux des anticorps A, B ou C précités dans des séries d'essais avec une succession diverse des anticorps cités, l'échantillon étant d'abord mis à réagir avec le premier anticorps de chaque série d'essais, puis le liquide surnageant n'ayant pas réagi est séparé et celui-ci est mis à réagir à chaque fois avec l'anticorps suivant de la série d'essais et, sur la base de la liaison antigène-anticorps existante de chaque série d'essais, on effectue un détermination qualitative et quantitative du composé (I).

3. Procédé selon les revendications 1 ou 2, caractérisé en que le composé répondant à la formule (I) est un cycle aromatique à six maillons substitué en méta, contenant jusqu'à trois atomes d'azote.

4. Procédé selon la revendication 3, caractérisé en ce que le composé (I) est une pyridine, une pyrimidine ou une triazine.

5. Procédé selon la revendication 2, caractérisé en ce que pour la détermination immunologique, on utilise trois anticorps A, B et C dans des séries d'essais, dans l'ordre suivant :
   1. première série d'essais : A B C
   2. deuxième série d'essais : B C A
   3. troisième série d'essais : C A B.

6. Procédé selon une ou plusieurs des revendications précédentes, caractérisé en ce que les anticorps sont des anticorps monoclonaux.

7. Procédé selon une ou plusieurs des revendications précédentes, caractérisé en ce qu'en plus du composé (I), on englobe également par réactivité croisée des composés analogues qui appartiennent à cette classe de composé et également des composés différents de celle-ci par leur structure, mais qui présentent la même activité biologique et un comportement à la liaison analogue.

8. Procédé selon une ou plusieurs des revendications précédentes, caractérisé en ce que la détection immunologique est effectuée à l'aide d'un isotope radioactif, d'une enzyme, d'une substance fluorescente ou d'une substance émettant un signal électrique.